(19) Europäisches Patentamt
European Patent Office
Office européen des brevets

(11) Veröffentlichungsnummer: **0 075 095**
**A2**

(12) # EUROPÄISCHE PATENTANMELDUNG

(21) Anmeldenummer: **82107150.3**

(22) Anmeldetag: **07.08.82**

(51) Int. Cl.³: **C 07 D 501/36,** C 07 D 498/04, A 61 K 31/545 // C07D249/12 ,(C07D498/04, 265/00, 205/00)

(30) Priorität: **23.09.81 CH 6138/81**
**29.07.82 CH 4598/82**

(43) Veröffentlichungstag der Anmeldung: **30.03.83 Patentblatt 83/13**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Montavon, Marc, Dr., Realpstrasse 72, CH-4054 Basel (CH)**
Erfinder: **Reiner, Roland, Dr., Rheinfelderstrasse 8, CH-4058 Basel (CH)**

(74) Vertreter: **Martin, Björn et al, Grenzacherstrasse 124 Postfach 3255, CH-4002 Basel (CH)**

(54) **Cephalosporinderivate, Verfahren zu deren Herstellung und Zwischenprodukte dafür sowie entsprechende pharmazeutische Präparate.**

(57) Leicht hydrolysierbare Ester von Cephalosporinderivaten der allgemeinen Formel

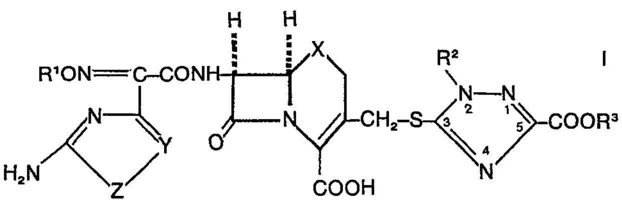

I

in der $R^1$ Wasserstoff, Methyl oder Carboxy-niederes Alkyl, X Schwefel, Sauerstoff oder eine der Gruppen –SO– und $-SO_2-$, Y die Gruppe –CH= oder Stickstoff, Z Schwefel oder Selen, $R^2$ gegebenenfalls durch Carboxy, Hydroxy, leicht hydrolysierbares Acyloxy oder Dimethylamino substituiertes niederes Alkyl oder Phenyl und $R^3$ niederes Alkyl, Phenyl-$C_{2-4}$-alkyl, $R^4$-Phenyl-niederes Alkyl, worin $R^4$ Halogen, niederes Alkyl oder niederes Alkoxy darstellt, bedeutet,

sowie Säureadditionssalze dieser Ester und Hydrate dieser Ester bzw. Säureadditionssalze, ferner Verfahren zu deren Herstellung und Zwischenprodukte dafür sowie entsprechende pharmazeutische Präparate.

Die Produkte sind antibiotisch, insbesondere bakterizid, wirksam.

EP 0 075 095 A2

0075095

F.Hoffmann-La Roche & Co.Aktiengesellschaft,Basel,Schweiz

RAN 4410/159

# Cephalosporinderivate, Verfahren zu deren Herstellung und Zwischenprodukte dafür sowie entsprechende pharmazeutische Präparate

Die vorliegende Erfindung betrifft neue Cephalosporinderivate und zwar leicht hydrolysierbare Ester von Verbindungen der allgemeinen Formel

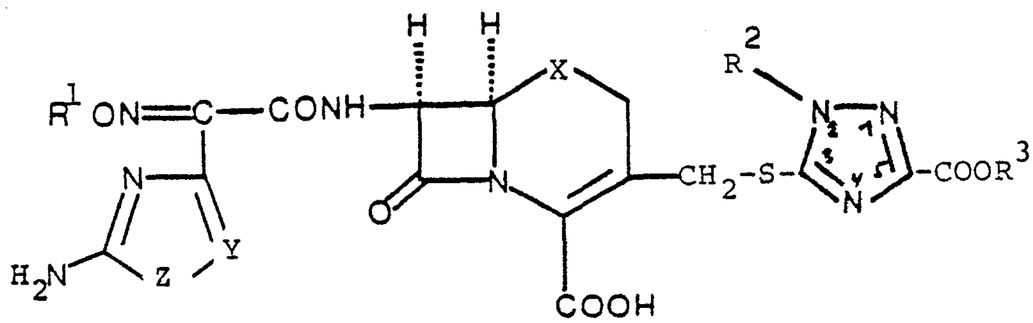

I

in der $R^1$ Wasserstoff, Methyl oder Carboxy-niederes Alkyl, X Schwefel, Sauerstoff oder eine der Gruppen -SO- und $-SO_2-$, Y die Gruppe -CH= oder Stickstoff, Z Schwefel oder Selen, $R^2$ gegebenenfalls durch Carboxy, Hydroxy, leicht hydrolysierbares Acyloxy oder Dimethylamino substituiertes niederes Alkyl oder Phenyl und $R^3$ niederes Alkyl, Phenyl-$C_{2-4}$-alkyl, $R^4$-Phenyl-niederes Alkyl, worin $R^4$ Halogen, niederes Alkyl oder

Mn/ 22.7.82

niederes Alkoxy darstellt, bedeutet, sowie Säureadditionssalze dieser Ester und Hydrate dieser Ester bzw. Säureadditionssalze.

Der Ausdruck "niederes Alkyl" allein oder in Zusammensetzungen stellt einen geradkettigen oder verzweigten niederen Alkylrest mit bis zu 7, vorzugsweise bis zu 4 Kohlenstoffatomen dar; z.B. Methyl, Aethyl, n-Propyl, Isopropyl, n-Butyl, Isobutyl, sec-Butyl, n-Pentyl, n-Heptyl. "Niederes Alkoxy" hat analoge Bedeutung. In den substituierten niederen Alkylresten kann sich der Substituent an beliebiger Stelle des Alkylrestes befinden, wie z.B. in 1-Carboxyäthyl, 2-Carboxyäthyl, 1-Carboxy-1-methyl-äthyl, 2-Carboxy-1-methyl-äthyl, 1-Carboxy-1-methyl-n-propyl, Carboxy-methyl. Ein bevorzugter Carboxy-nieder-Alkylrest ist 1-Carboxy-1-methyl-äthyl. Durch leicht hydrolysierbares Acyloxy substituiertes Alkyl kann z.B. Pivaloyloxymethyl, Acetoxymethyl, 1-(Pivaloyloxy)-äthyl, 1-(Aethoxycarbonyloxy)-äthyl und dgl. darstellen. "Halogen" bedeutet vorzugsweise Chlor, Brom oder Fluor.

Als leicht hydrolysierbare Ester der Verbindungen der Formel I sind Verbindungen der Formel I zu verstehen, worin mindestens eine Carboxygruppe in Form einer leicht hydrolysierbaren Estergruppe vorliegt. Beispiele solcher Ester sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxy-nieder-alkylester, z.B. der Methoxymethylester; der gegebenenfalls durch Halogen, niederes Alkyl oder niederes Alkoxy substituierte Phenylester; und die niederen Alkanoylaminomethylester, z.B. der Acetamidomethylester. Auch andere Ester, z.B. die Benzyl- und Cyanmethylester können brauchbar sein. Die Ester können Mono-, Di- oder Tri-ester sein. Esterbildung

kann in Verbindung mit dem 4-Carboxyrest bzw. einer Carboxy-nieder-alkylgruppe $R^2$ sowie mit einer Carboxy-nieder-alkylgruppe $R^1$ auftreten.

Die leicht hydrolysierbaren Ester der Verbindungen der Formel I bilden Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Monoaryl-sulfonate, wie Äethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die leicht hydrolysierbaren Ester der Verbindungen der Formel I sowie deren Säureadditionssalze können hydratisiert sein. Die Hydratisierung kann im Zuge des Herstellungsverfahren erfolgen oder allmählich als Folge hygroskopischer Eigenschaften eines zunächst wasserfreien Produktes auftreten.

Die erfindungsgemässen Produkte können in der syn-isomeren Form

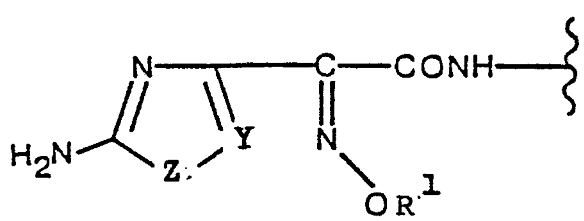

oder in der anti-isomeren Form

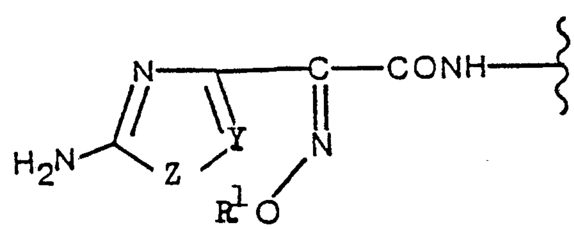

bzw. als Gemische dieser beiden Formen vorliegen. Bevorzugt ist die syn-isomere Form bzw. Gemische, in denen die syn-isomere Form überwiegt.

Die obigen Cephalosporinderivate können erfindungsgemäss dadurch hergestellt werden, dass man die entsprechende Carbonsäure, d.h. eine Verbindung der Formel I oder ein Salz dieser Verbindung einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

Die im obigen Verfahren verwendete Carbonsäure der Formel I kann z.B. dadurch hergestellt werden, dass man

a) zur Herstellung einer Carbonsäure der Formel I, worin $R^1$ Wasserstoff oder Methyl und Y die Gruppe -CH= darstellt, eine Verbindung der allgemeinen Formel

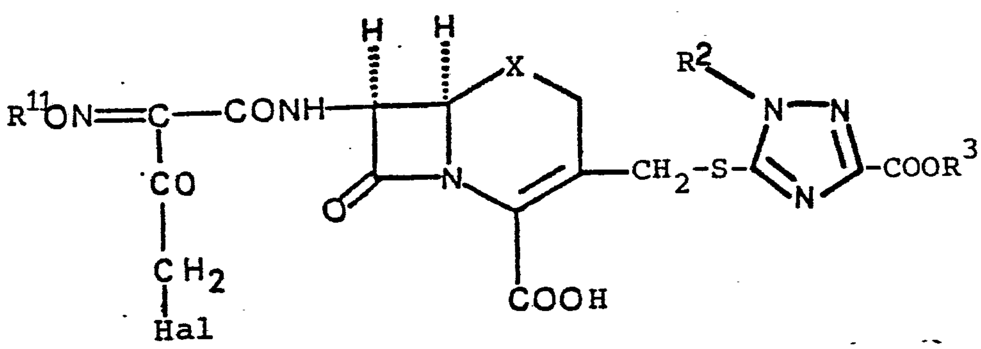

II

in der $R^2$, $R^3$ und X die oben gegebene Bedeutung haben, $R^{11}$ Wasserstoff oder Methyl, Hal ein Halogenatom darstellt und die Carboxygruppe in geschützter Form vorliegen kann,

oder ein Salz dieser Verbindung mit Thioharnstoff oder Selenharnstoff umsetzt und gegebenenfalls eine allenfalls vorhandene Carboxyschutzgruppe abspaltet, oder dass man

b) aus einer Verbindung der allgemeinen Formel

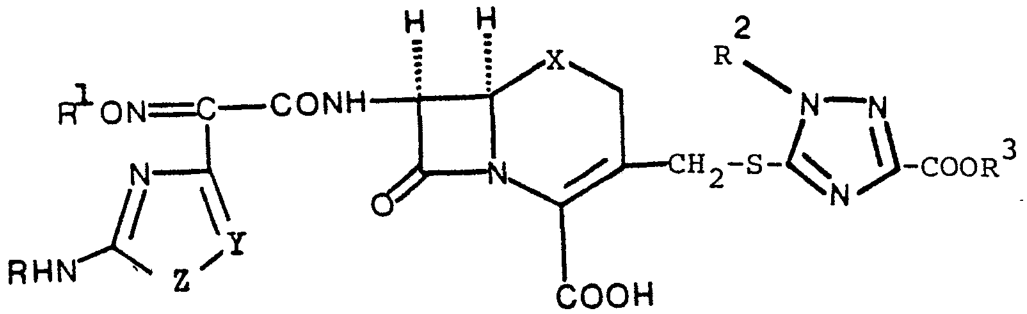

III

in der $R^1$, $R^2$, $R^3$, X, Y und Z die oben gegebene Bedeutung haben, R eine abspaltbare Schutzgruppe darstellt und die Carboxygruppe in geschützter Form vorliegen kann,

oder aus einem Salz dieser Verbindung die Schutzgruppe R und gegebenenfalls eine allenfalls vorhandene Carboxyschutzgruppe abspaltet, oder dass man

c) eine Verbindung der allgemeinen Formel

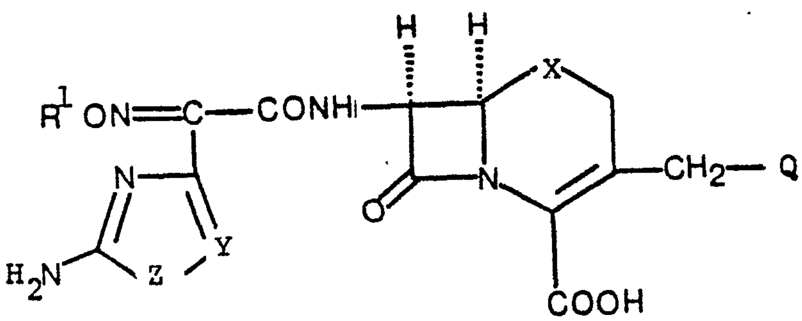

IV

in der $R^1$, X, Y und Z die oben gegebene Bedeutung haben, Q eine austretende Gruppe darstellt und die Carboxygruppe durch Salzbildung mit einer anorganischen oder tertiären organischen Base geschützt sein kann,

oder ein Salz dieser Verbindung in Gegenwart von Wasser mit einem Thiol der allgemeinen Formel

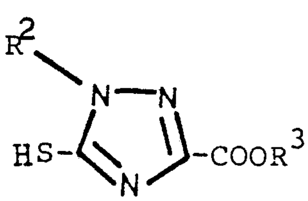

V

in der $R^2$ und $R^3$ die oben gegebene Bedeutung haben, umsetzt, und gegebenenfalls eine allenfalls vorhandene Carboxyschutzgruppe abspaltet, oder dass man

d) eine Verbindung der allgemeinen Formel

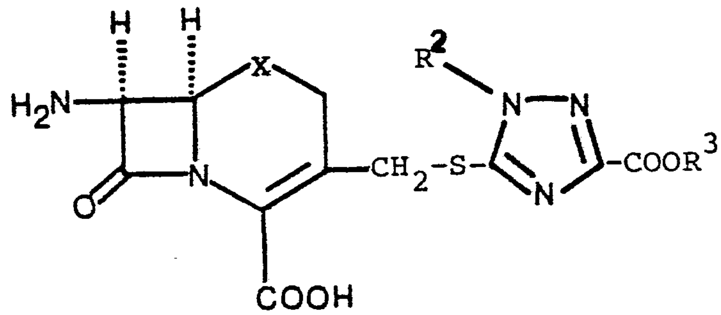

VI

in der X, $R^2$ und $R^3$ die oben gegebene Bedeutung haben, und die Carboxygruppe und/oder Aminogruppe in geschützter Form vorliegen kann,

mit einem aktivierten Thioester der Formel

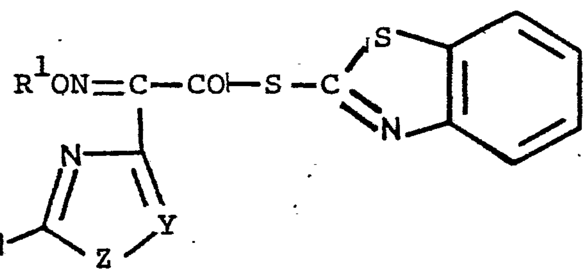

VII

in der $R^1$, Y und Z die oben angegebene Bedeutung haben, umsetzt und gegebenenfalls eine allenfalls vorhandene Carboxyschutzgruppe abspaltet, oder dass man

e) zur Herstellung von Verbindungen der Formel I, worin X eine der Gruppen -SO- und $-SO_2-$ darstellt, eine Verbindung der Formel I, worin X Schwefel oder die Gruppe -SO- darstellt, oder ein Salz dieser Verbindung oxidiert.

Die in den Ausgangsverbindungen der Formeln II und III vorhandene Carboxygruppe in 3-Stellung kann wahlweise geschützt sein, z.B. durch Veresterung zu einem leicht

spaltbaren Ester, wie dem Silylester, z.B. dem Trimethylsilylester. Die Carboxygruppe kann auch durch Salzbildung mit einer anorganischen oder tertiären organischen Base, wie Triäthylamin, geschützt werden.

Das erfindungsgemäss eingesetzte Halogenid der Formel II kann, wenn $R^{11}$ Methyl darstellt, z.B. durch Umsetzung einer Verbindung der Formel VI mit einer halogenierten Carbonsäure der allgemeinen Formel

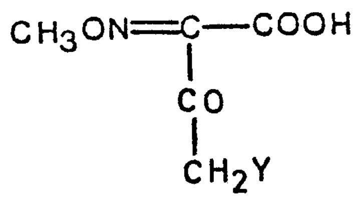

XV

in der Y ein Halogenatom darstellt, oder mit einem reaktionsfähigen Derivat dieser Verbindung hergestellt werden. Die halogenierte Carbonsäure der Formel XV wird entweder in freier Form eingesetzt in Gegenwart eines Kondensationsmittels, z.B. eines N,N'-disubstituierten Carbodiimides, wie N,N'-Dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z.B. mit Monomethyl- oder Moniisopropylcarbonat, oder in Form eines aktivierten Esters, wie des p-Nitrophenylesters, 2,4-Dinitrophenylesters, N-Hydroxysuccinimidesters oder N-Hydroxyphthalimidesters. Die Reaktion erfolgt im allgemeinen in einem inerten organischen Lösungsmittel, z.B. in einem halogenierten Kohlenwasserstoff, wie Chloroform, Dichlormethan, Tetrachlorkohlenstoff, in einem Aether, z.B. Tetrahydrofuran, Dioxan, in Dimethylformamid, Dimethylacetamid, Wasser oder Mischungen davon. Die Reaktionstemperatur liegt vornehmlich im Bereich von etwa -50 bis +40°C, vorzugsweise bei etwa -10 bis +10°C.

Halogenide der Formel II, worin $R^{11}$ Wasserstoff darstellt, können hergestellt werden, indem man eine Verbindung der Formel VI mit Diketen und dem entsprechenden Halogen

(Brom oder Chlor) in das entsprechende 4-Halogenaceto-acetamidoderivat der allgemeinen Formel

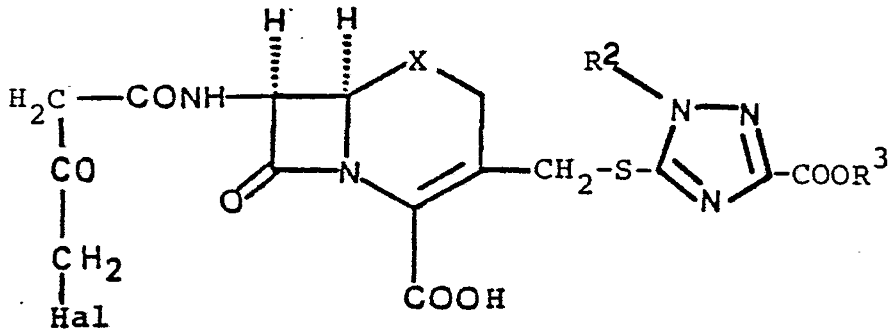

XVI

worin X, $R^2$ und $R^3$ und Hal die oben gegebene Bedeutung haben, überführt und dieses Produkt mit einem Nitrosierungsmittel behandelt.

Die erfindungsgemässe Umsetzung des Halogenids der Formel II bzw. eines Salzes davon mit Thioharnstoff oder Selenharnstoff gemäss obiger Variante a) erfolgt vorzugsweise in einem inerten Lösungsmittel, wie z.B. in einem niederen Alkanol, z.B. Aethanol, in einem niederen Keton, wie Aceton, in einem Aether, wie Tetrahydrofuran oder Dioxan, in Dimethylformamid, Dimethylacetamid, in Wasser oder in Mischungen davon. Die Reaktionstemperatur liegt im allgemeinen im Bereich von etwa 0°C bis 60°C, vorzugsweise wird bei Zimmertemperatur gearbeitet. Als Halogenid der Formel II kann das Chlorid, Bromid, Fluorid oder Jodid eingesetzt werden, bevorzugt verwendet man das Chlorid oder das Bromid. Es kann die freie Säure der Formel II eingesetzt werden, wahlweise aber auch ein Salz davon, wobei die gleichen Salze wie die oben erläuterten Salze der Verbindungen der Formel I in Betracht kommen.

Nach Durchführung der Verfahrensvariante a) kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Wenn die Carboxylgruppe durch Salzbildung (z.B. mit Triäthylamin) geschützt ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Die Schutzgruppen R in den für die Variante b) einsetzbaren Ausgangsverbindungen der Formel III sind beispielsweise sauer-hydrolytisch abspaltbare Schutzgruppen, wie z.B. t-Butoxycarbonyl oder Trityl, oder auch basischhydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Die Ausgangsverbindungen der Formel III können z.B. durch N-Acylierung der entsprechenden 7-Aminoverbindung hergestellt werden, und zwar indem man eine Verbindung der obigen Formel VI mit einer Säure der allgemeinen Formel

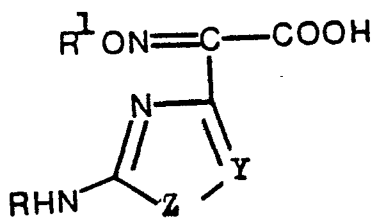

VIII

in der R, $R^1$, Y und Z die oben gegebene Bedeutung haben,

oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel VI vorhandene Carboxygruppe kann wahlweise geschützt sein, und zwar in der oben für die herzustellenden Ausgangsverbindungen der Formeln II und III erläuterten Weise. Die Aminogruppe der Verbindung der Formel III kann z.B. durch eine Silylschutzgruppe, wie Trimethylsilyl, geschützt sein.

Als reaktionsfähige funktionelle Derivate von Säuren

der Formel VIII kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel VI mit der Säure der Formel VIII oder einem reaktionsfähigen funktionellen Derivat davon kann in an sich bekannter Weise durchgeführt werden. So kann man z.B. eine freie Säure der Formel VIII mit einem der erwähnten Ester entsprechend Formel VI mittels eines Carbodiimids, wie Dicyclohexylcarbodiimid, in einem inerten Lösungsmittel, wie Aethylacetat, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel VI, z.B. ein Trialkylammoniumsalz, wie das Triäthylammoniumsalz, mit einem reaktionsfähigen funktionellen Derivat einer Säure der Formel VIII wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel VIII mit dem Amin der Formel VI umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, gegebenenfalls im Gemisch mit einem inerten organischen Lösungsmittel, wie Tetrahydrofuran oder Dioxan. Es kann auch in einem aprotischen organischen Lösungsmittel, wie z.B. Dimethyl-

formamid, Dimethylsulfoxid oder Hexamethylphosphorsäure-triamid, gearbeitet werden. Bei Verwendung von silylierten Ausgangsverbindungen der Formel VI wird in wasserfreiem Medium gearbeitet.

Die Umsetzung der 7-Aminoverbindung der Formel VI mit der Säure der Formel VIII oder einem reaktionsfähigen funktionellen Derivat davon, kann zweckmässig bei Temperaturen zwischen etwa -40°C und Zimmertemperatur, beispielsweise bei etwa 0-10°C, erfolgen.

Ausgangsverbindungen der Formel III, worin R nicht monohalogeniert ist, wie in Brom-, Chlor- oder Jodacetyl, können auch durch Thiolierung hergestellt werden, und zwar indem man eine Verbindung der allgemeinen Formel

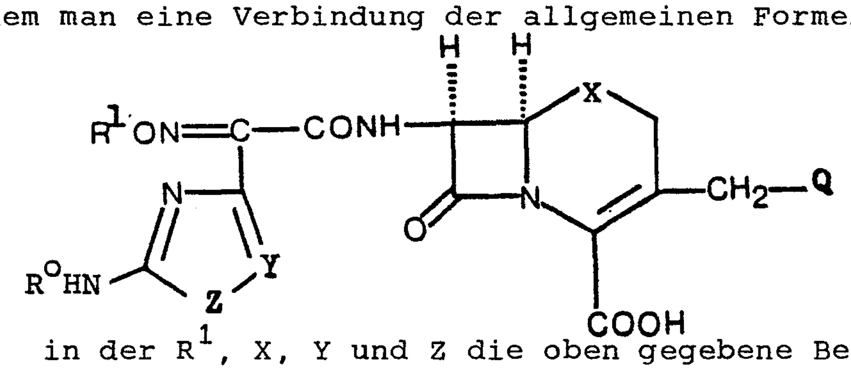

IX

in der $R^1$, X, Y und Z die oben gegebene Bedeutung haben, $R^o$ wie R ist, jedoch nicht monohalogeniert sein kann, Q eine austretende Gruppe darstellt und die Carboxygruppe durch Salzbildung mit einer anorganischen oder tertiären organischen Base geschützt sein kann,

in Gegenwart von Wasser mit einem Thiol der Formel V umsetzt und eine allenfalls vorhandene Carboxyschutzgruppe gegebenenfalls abspaltet.

Als austretende Gruppe Q kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel IX mit dem Thiol der Formel V kann in an sich bekannter Weise, z.B.

bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden. Die Carboxygruppe der erhaltenen Verbindung III kann erwünschtenfalls geschützt werden, z.B. durch Salzbildung oder Veresterung.

7-Aminoverbindungen der Formel VI können ausgehend von einer Verbindung der Formel

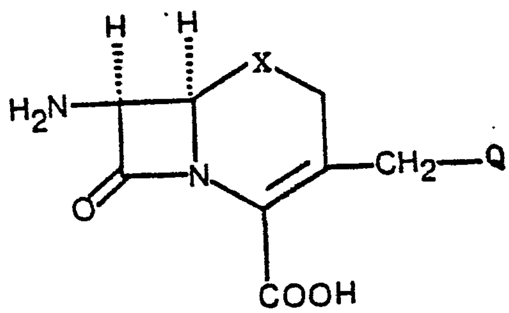

XI

in der X die oben gegebene Bedeutung hat, Q eine austretende Gruppe darstellt und die Carboxygruppe durch Salzbildung mit einer anorganischen oder tertiären organischen Base geschützt sein kann,

in Gegenwart von Wasser durch Umsetzung mit einem Thiol der Formel V hergestellt werden. Die Umsetzung kann unter den gleichen Bedingungen wie denjenigen erfolgen, wie sie für die Umsetzung der Ausgangsverbindungen IX mit V beschrieben wurden. Andererseits können die Verbindungen der Formel IX ausgehend von einer Verbindung der Formel XI und einer Säure der Formel VIII oder einem reaktionsfähigen funktionellen Derivat davon unter den gleichen Bedingungen hergestellt werden, wie sie für die Umsetzung der Verbindungen der Formeln VI und VIII beschrieben wurden.

Die Carboxygruppe und/oder die Aminogruppe der erhaltenen Verbindung VI können erwünschtenfalls geschützt werden, z.B. durch Veresterung oder Salzbildung der Carboxygruppe oder durch Silylierung.

Säuren der Formel VIII mit Y= -CH= und Z = Se können z.B. durch Umsetzung einer Verbindung der allgemeinen Formel

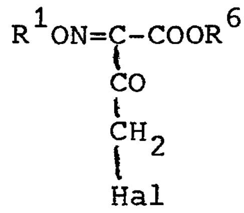

XII

in der $R^1$ und Hal die oben gegebene Bedeutung haben und $R^6$ niederes Alkyl, insbesondere Methyl oder Aethyl, darstellt,

mit Selenharnstoff hergestellt werden, in der gleichen Weise wie oben für die Umsetzung der Ausgangsverbindungen der Formel II mit Selenharnstoff beschrieben. Die so erhaltene Verbindung der Formel

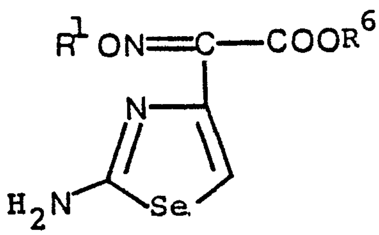

XIII

worin $R^1$ und $R^6$ die oben gegebene Bedeutung haben, wird anschliessend an der Aminogruppe geschützt, vorzugsweise durch Umsetzung mit dem entsprechenden Halogenid der Formel R-Hal, und die erhaltene Verbindung der Formel

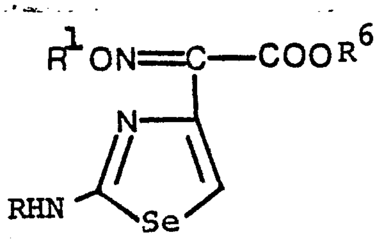

XIV

worin R, $R^1$ und $R^6$ die oben gegebene Bedeutung haben, zur Abspaltung der Estergruppe $R^6$ sauer oder alkalisch verseift. Die reaktionsfähigen Derivate der so erhaltenen Säuren der Formel VIII werden in an sich bekannter Weise hergestellt.

Die Säuren der Formel VIII mit Y = Stickstoff können gemäss EP 27599 bzw. (wenn Z = Selen) in Analogie dazu hergestellt werden.

Die Thiole der Formel V stehen im tautomeren Gleichgewicht mit den entsprechenden Thionen und können in an sich bekannter Weise hergestellt werden , beispielsweise aus einem 2-$R^2$-Thiosemicarbazid durch Kochen mit einem Alkalimetall-nieder-alkanolat, z.B. Natriummethylat in einem niederen Alkanol, wie Methanol, und einem Oxalsäure-di-$R^3$-ester. Wahlweise wird das 2-$R^2$-Thiosemicarbazid in einem inerten Lösungsmittel, wie Acetonitril, mit dem entsprechenden Oxalsäure-mono-$R^3$-ester-säurehalogenid, z.B. dem -säurechlorid, in Gegenwart eines säurebindenden Mittels erhitzt und das Kondensationsprodukt anschliessend wie oben mit dem Alkalimetall-niederalkanolat im niederen Alkanol cyclisiert. Der erhaltene 4,5-Dihydro-1-$R^2$-5-thioxo-1H-1,2,4-triazol-3-carbonsäure-$R^3$-ester kann erwünschtenfalls verseift und wieder umgeestert werden.

Gemäss Verfahrensvariante b) wird die Aminoschutzgruppe R einer Ausgangsverbindung der Formel III abgespalten. Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa 0°C bis +40°C. Alkalisch abspaltbare Schutzgruppen werden im allgemeinen mit verdünnter wässriger Lauge bei 0°C bis 30°C hydrolysiert. Die Chloracetyl-, Bromacetyl- und Jodacetyl-Schutzgruppen können mittels Thioharnstoff in saurem, neutralem oder alkalischem Milieu bei etwa 0-30°C abgespalten werden. Hydrogenolytische Abspaltung (z.B. Abspaltung von Benzyl) ist ungeeignet, da bei der Hydrogenolyse die Oximfunktion zur Aminogruppe reduziert wird.

Die allenfalls vorhandene Carboxyschutzgruppe kann in der gleichen Weise wie oben für die Verfahrensvariante a) beschrieben abgespalten werden. Die Carboxyschutzgruppe kann in der gleichen Weise auch vor der Abspaltung der

Schutzgruppe R abgespalten werden.

Die für die Verfahrensvariante c) einsetzbaren Ausgangsverbindungen der allgemeinen Formel IV können durch Abspaltung der Aminoschutzgruppe $R^{O}$ der oben beschriebenen Verbindungen der Formel IX erhalten werden. Diese Abspaltung kann in der gleichen Weise durchgeführt werden wie die oben beschriebene Abspaltung der Aminoschutzgruppen R der Ausgangsverbindungen der Formel III.

Als austretende Gruppe Q einer Verbindung der Formel IV kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel IV mit dem Thiol der Formel V kann in an sich bekannter Weise, z.B. bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden. Die allenfalls vorhandene Carboxyschutzgruppe kann in der gleichen Weise wie oben für die Verfahrensvariante a) beschrieben abgespalten werden.

Der für die Variante d) verwendete aktivierte Ester VII kann aus der Carbonsäure VIII (gegebenenfalls geschützt wie oben erläutert) durch Umsetzung mit 2,2'-Dithiobisbenzothiazol in Gegenwart von Triphenylphosphin oder eines Tri-niederalkylphosphits bei Raumtemperatur in einem inerten organischen Lösungsmittel, wie z.B. Acetonitril oder Methylenchlorid, hergestellt werden. Die anschliessende Umsetzung der Verbindungen VI und VII wird vorzugsweise in einem inerten Lösungsmittel oder im Gemisch mit Wasser, z.B. in Methylenchlorid oder Aethylacetat, bei etwa Raumtemperatur, vorzugsweise in Gegenwart einer Base, wie Triäthylamin, ausgeführt.

Eine allenfalls noch vorhandene Carboxyschutzgruppe

kann anschliessend, wie oben für die Verfahrensvariante a) bereits erwähnt, abgespalten werden.

Die Variante e), die Oxidation von Verbindungen der Formel I, worin X Schwefel oder die Gruppe -SO- darstellt, bzw. eines Salzes davon, erfolgt durch Behandlung mit einem organischen oder anorganischen Oxidationsmittel. Als Oxidationsmittel dienen verschiedene, Sauerstoff leicht abgebende Verbindungen, wie z.B. organische Peroxide, z.B. monosubstituierte organische Peroxide, wie $C_1$-$C_4$ Alkyl- oder Alkanoylhydroperoxide, wie t-Butylhydroperoxid, Perameisensäure, Peressigsäure; sowie phenylsubstituierte Derivate dieser Hydroperoxide, wie Cumolhydroperoxid, Perbenzoesäure. Der Phenylsubstituent kann gegebenenfalls eine weitere niedere Gruppe, z.B. $C_1$-$C_4$-Alkyl oder -Alkoxy, Halogen oder Carboxygruppe tragen, z.B. 4-Methylperbenzoesäure, 4-Methoxyperbenzoesäure, 3-Chlorperbenzoesäure, Monoperphthalsäure. Als Oxidationsmittel können auch verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol, Aethanol, oder einer niederen, gegebenenfalls halogenierten Alkancarbonsäure, z.B. Ameisensäure, Essigsäure, Trifluoressigsäure. Die Reaktionstemperatur bewegt sich vornehmlich im Bereiche von -20°C bis +50°C.

Bei Verwendung von äquimolaren Mengen Oxidationsmittel bzw. leichtem Ueberschuss im Verhältnis zur Ausgangsverbindung der Formel I, worin X Schwefel darstellt, bzw. einem Salz davon, erhält man vornehmlich das entsprechende Sulfoxid der Formel I, worin X die Gruppe -SO- darstellt. Erhöht man die Menge des Oxidationsmittels auf das doppelte stöchiometrische Verhältnis oder mehr, bildet sich das entsprechende Sulfon der Formel I, worin X die Gruppe $-SO_2-$ darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Die erhaltene Carbonsäure der Formel I kann anschliessend in ein Salz übergeführt werden, z.B. in ein Säureadditionssalz wie oben beschrieben, oder auch in Salze mit Basen, z.B. Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Mono-, Di- oder Trisalze sein. Die Salze werden in an sich bekannter Weise hergestellt, z.B. durch Umsetzen der Carbonsäure der Formel I mit einer äquivalenten Menge der gewünschten Säure bzw. Base, zweckmässig in einem Lösungsmittel, wie Wasser oder in einem organischen Lösungsmittel, wie Aethanol, Methanol, Aceton und anderen mehr. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Zur erfindungsgemässen Herstellung der leicht hydrolysierbaren (Mono-, Di- oder Tri-)Ester der Carbonsäuren der Formel I wird die Carbonsäure bzw. eines ihrer Salze vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Je nach der verwendeten Menge Veresterungsmittel erhält man Mono-, Di- oder Triester. Für die vollständige Veresterung einer Säure der Formel I verwendet man vorzugsweise einen Ueberschuss an dem entsprechenden Halogenid. Die Veresterungsreaktion wird vorzugsweise in einem inerten organischen Lösungsmittel, gegebenenfalls im Gemisch mit Wasser, durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Ein erhaltener Ester kann erwünschtenfalls S-oxidiert werden, wobei die Gruppe X = Schwefel gegen -SO- bzw. $-SO_2-$ ausgetauscht wird. Die Oxidation kann in der oben beschriebenen Weise erfolgen.

Die Säureadditionssalze und Hydrate der Ester der Verbindungen der Formel I bzw. der Hydrate dieser Säureadditionssalze können in an sich bekannter Weise hergestellt werden, die Säureadditionssalze z.B. durch Umsetzung eines Esters einer Carbonsäure der Formel I mit einer äquivalenten Menge der gewünschten Säure, zweckmässig in einem Lösungsmittel, wie Wasser oder in einem organischen Lösungsmittel, wie Aethanol, Methanol, Aceton und anderem mehr. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt einer feuchten Atmosphäre, z.B. bei etwa +10°C bis +40°C, ausgesetzt werden.

Ein allenfalls erhaltenes syn/anti-Gemisch kann in die

entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches. Vorzugsweise erhält man aber die erfindungsgemässen Ester in der syn-isomeren Form, indem man eine Ausgangsverbindung der Formel IV in syn-Form einsetzt.

Die erfindungsgemässen Ester der Carbonsäuren der Formel I sowie diese Carbonsäuren selbst und die entsprechenden Salze bzw. die Hydrate dieser Produkte sind antibiotisch, insbesondere bakterizid wirksam. Sie besitzen ein breites Wirkungsspektrum gegen Gram-positive Bakterien, z.B. Streptokokken, und gegen Gram-negative Bakterien, einschliesslich β-Lactamase-bildende Gram-negative Erreger, wie Escherichia coli, Serratia marcescens und Klebsiella pneumoniae.

Die erfindungsgemässen Ester der Carbonsäuren der Formel I sowie diese Carbonsäuren selbst und die entsprechenden Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 4 g, insbesondere etwa 0,25 g bis etwa 2 g in Betracht. Enterale oder parenterale Verabreichung ist möglich; die leicht hydrolysierbaren Ester der Verbindungen der Formel I sowie die entsprechenden Säureadditionssalze und Hydrate besitzen den besonderen Vorzug der Verwendbarkeit für die enterale, z.B. die orale, Verabreichung.

Die orale antimikrobielle Wirksamkeit der erwähnten Produkte kann anhand von in-vivo-Versuchen an der Maus nachgewiesen werden. Im nachstehenden bedeutet die kurative Dosis ($CD_{50}$, p.o., mg/kg) diejenige perorale Dosis, bei der 50% der getesteten Mäuse überleben.

Testverbindungen:

Produkt A: Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carboxylat-pivalat

Produkt B: Methylen-(6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carboxylat-pivalat

Produkt C: Methylen-(6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carboxylat-pivalat

Produkt D: Methylen-(6R,7R)-7-[(Z)-2-(2-Amino-4-thiazolyl)-2-(methoxyimino)-acetamido]-3-[[[5-(methoxycarbonyl)-2-[[[(pivaloyloxy)methoxy]-carbonyl]methyl]-2H-1,2,4-triazol-3-yl]thio]-methyl]-8-oxo-5-thia-1-azabicyclo[4.2.0]oct-2-en-2-carboxylat-pivalat

Produkt E: Methylen-(6R,7R)-7-[(Z)-2-(2-Amino-4-thiazolyl)-2-(methoxyimino)-acetamido]-3-[[[5-(methoxycarbonyl)-2-(n-propyl)-2H-1,2,4-triazol-3-yl]thio]methyl]-8-oxo-5-thia-1-azabicyclo-[4.2.0]oct-2-en-2-carboxylat-pivalat

Produkt F: Methylen-(6R,7R)-7-[(Z)-2-(2-Amino-4-thiazolyl)-2-(methoxyimino)-acetamido]-3-[[[5-(methoxycarbonyl)-2-phenyl-2H-1,2,4-triazol-3-yl]thio]methyl]-8-oxo-5-thia-1-azabicyclo-[4.2.0]oct-2-en-2-carboxylat-pivalat

- Cephalexin (vorbekanntes, anerkannt oral wirksames Cephalosporin)

Ergebnis ($CD_{50}$, p.o., mg/kg)

| Erreger | Produkte | | | | | | |
| --- | --- | --- | --- | --- | --- | --- | --- |
| | A | B | C | D | E | F | Cephalexin |
| Escherichia coli | 0,07 | | | | 0,04 | | 3,2 |
| Serratia marcescens | 1,5 | 0,8 | 2,54 | >6 | 1,8 | 5,8 | >50 |
| Klebsiella pneumoniae | 3,7 | | | 12,5 | 2,5 | >12 | |
| Streptococcus pyogenes | 0,72 | 2,1 | 0,53 | 8,8 | 0,89 | 1,5 | 1,8 |

Die Produkte A, B, D, E und F sind mindestens so ungiftig wie Cephalexin ($LD_{50}$ p.o. an der Maus nach 24 Stunden: >5000 mg/kg für die Produkte A, B, D, E und F; 1600-4500 mg/kg für Cephalexin).

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten.

Beispiel 1

Herstellung von Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carboxylat-pivalat.

5,9 g Natriumsalz der (6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure werden in 100 ml Dimethylformamid gelöst und bei 0-5°C mit 4,48 g Pivaloyloxymethyljodid versetzt. Das Gemisch wird 30 Minuten bei 0-5°C unter Stickstoff-Begasung gerührt. Dann wird es auf 500 ml Wasser gegossen und 2mal mit je 300 ml Aethylacetat extrahiert. Die vereinigten organischen Phasen werden nacheinander 2mal mit 200 ml Wasser, 2mal mit 250 ml 5%iger wässriger Natriumhydrogencarbonat-Lösung und nochmals mit 250 ml Wasser gewaschen, mit Natriumsulfat getrocknet und im Vakuum bei 40°C eingeengt. Nach dem Versetzen mit tiefsiedendem Petroläther wird das ausgefallene Material abgenutscht, mit tiefsiedendem Petroläther gewaschen und über Nacht im Vakuum bei 20°C getrocknet. Man erhält ein beiges Rohprodukt, welches, zwecks Reinigung, an einer Kieselgelsäule mit Aethylacetat als Elutionsmittel chromatographiert wird. Man erhält weisse Titelsubstanz mit $[\alpha]_D^{25}$ = -166,4° (c = 1,507 in Aceton). Die Mikroanalyse und das Kernresonanzspektrum entsprechen der angegebenen Struktur.

Das oben verwendete Natriumsalz der (6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure kann wie folgt hergestellt werden:

a) Herstellung von 4,5-Dihydro-1-methyl-5-thioxo-1H-1,2,4-triazol-3-carbonsäure-methylester

12,9 g 1-Methoxyoxalyl-2-methyl-thiosemicarbazid werden einer Lösung von 1,6 g Natrium in 200 ml Methanol zugegeben. Das Gemisch wird 4 Stunden unter Rückfluss gekocht. Die nach einer 1/2 Stunde ausgefallene Substanz wird abgetrennt. Die Mutterlauge wird im Vakuum bei 40°C eingedampft. Der Eindampfrückstand wird in 100 ml Wasser gelöst und mit konz. Salzsäure angesäuert. Das dabei ausgeschiedene Kristallisat liefert nach dem Umkristallisieren aus Wasser reinen, farblosen 4,5-Dihydro-1-methyl-5-thioxo-1H-1,2,4-triazol-3-carbonsäure-methylester vom Schmelzpunkt 188-190°C (Zers.).

b) Herstellung von (6R,7R)-7-Amino-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure

27,2 g 7-Aminocephalosporinsäure werden zusammen mit 18,2 g 4,5-Dihydro-1-methyl-5-thioxo-1H-1,2,4-triazol-3-carbonsäure-methylester in 250 ml Acetonitril suspendiert. Zu dieser Suspension werden 42,6 g Bortrifluorid-ätherat hinzugegeben. Das Reaktionsgemisch wird 30 Minuten bei 50-55°C unter Stickstoff-Begasung und Feuchtigkeitsausschluss gerührt. Dann wird es auf 20°C gekühlt, mit 550 ml Wasser versetzt und das pH mit konz. wässrigem Ammoniak auf 3,5 gestellt. Das Gemisch wird 30 Minuten bei 0-5°C gerührt. Das ausgefallene Material wird abgenutscht, nacheinander mit 500 ml Wasser, 300 ml Methanol, 300 ml Aceton und 300 ml tiefsiedendem Petroläther gewaschen und über Nacht im Vakuum bei 45°C getrocknet. Man erhält reine beige Titelsubstanz mit einem der angegebenen Struktur entsprechenden Kernresonanzspektrum.

c) Herstellung des Trimethylsilylesters der (6R,7R)-7-Amino-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure

23,12 g (6R,7R)-7-Amino-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure werden in 480 ml Aethylacetat suspendiert und mit 60 ml N,O-Bis-(trimethylsilyl)-acetamid versetzt. Es entsteht sofort eine orange Lösung. Diese wird auf -10°C gekühlt und bei dieser Temperatur bis zur Acylierung aufbewahrt.

d) Herstellung von 4-Brom-2-(Z)-methoxyimino-3-oxo-buttersäure-chlorid

13,44 g 4-Brom-2-(Z)-methoxyimino-3-oxo-buttersäure werden in 180 ml Methylenchlorid gelöst und bei 0-5°C mit 12,48 g Phosphorpentachlorid versetzt. Das Reaktionsgemisch wird 15 Minuten bei 0-5°C und 45 Minuten ohne Kühlung gerührt.

e) Herstellung von (6R,7R)-7-[4-Brom-2-(Z)-methoxyimino-3-oxo-butyramido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure

Die unter d) hergestellte Säurechloridlösung wird während ca. 15 Minuten bei -10-0°C zur unter c) hergestellten Silylesterlösung getropft. Das Reaktionsgemisch wird 1 Stunde bei 0-5°C und 1 Stunde bei 20°C gerührt. Dann wird es mit 500 ml Aethylacetat und 250 ml Wasser unter Rühren versetzt. Die wässrige Phase wird abgetrennt und die gelbe organische Phase noch 3mal mit je 500 ml Wasser gewaschen, mit Natriumsulfat getrocknet und im Vakuum bei 40°C stark eingeengt. Nach dem Versetzen mit Aether wird das ausgefallene Material abgenutscht, mit Aether und tiefsiedendem Petroläther gewaschen und im Vakuum bei 20°C getrocknet. Die Substanz wird noch 1mal aus Aethylacetat/Aether umgefällt. Man erhält reine weisse, amorphe Titelsubstanz mit $[\alpha]_D^{25} = -129,6°$ (c = 1 in Aceton). Das Kernresonanzspektrum entspricht der angegebenen Struktur.

f) Herstellung des Natriumsalzes der (6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure

12,42 g (6R,7R)-7-[4-Brom-2-(Z)-methoxyimino-3-oxo-butyramido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carbonsäure werden in einem Gemisch von 170 ml Aethanol und 170 ml Methanol gelöst und mit 3,15 g Thioharnstoff versetzt. Das Reaktionsgemisch wird 45 Minuten bei 20°C gerührt, mit Methanol auf 800 ml verdünnt und mit 30 ml einer 2N Lösung von 2-Aethylcapronsäure-Natriumsalz in Aethylester versetzt. Dem Gemisch werden 5 g Aktivkohle zugegeben und es wird weitere 30 Minuten bei 20°C gerührt. Dann wird es zwecks Abtrennen der Kohle durch einen Faltenfilter filtriert. Das hellgelbe Filtrat wird mit 200 ml Aethanol versetzt und im Vakuum bei 40°C stark eingeengt. Das dabei ausgefallene Material wird abgenutscht, mit Aethanol und tiefsiedendem Petroläther gewaschen und über Nacht im Hochvakuum bei 45°C getrocknet. Man erhält reine Titelsubstanz mit $[\alpha]_D^{25}$ = -35,9° (c = 1 in Wasser). Die Mikroanalyse und das Kernresonanzspektrum entsprechen der angegebenen Struktur.

## Beispiel 2

Herstellung von Methylen-(6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carboxylat-pivalat

5 g Natriumsalz der (6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carbonsäure werden in 80 ml Dimethylformamid gelöst und bei 0-5°C mit 3,7 g Pivaloyloxymethyljodid versetzt. Das Gemisch wird 1 Stunde bei 0-5°C gerührt, anschliessend auf 500 ml Wasser gegossen und 2mal mit 250 ml Aethylacetat extrahiert. Die vereinigten organischen Phasen werden nacheinander mit 250 ml Wasser, 2mal mit 250 ml 5%iger wässriger Natriumhydrogencarbonat-Lösung und nochmals mit 250 ml Wasser gewaschen, über Natriumsulfat getrocknet und im Vakuum bei 40°C stark eingeengt. Nach Zugabe von tiefsiedendem Petroläther wird das rohe, amorph ausgefallene Material abgenutscht, mit tiefsiedendem Petroläther gewaschen und über Nacht im Vakuum bei 20°C getrocknet. Man erhält ein beige-farbenes Rohprodukt, welches, zwecks Reinigung, einer Flash-Chromatographie an einer Säule mit Kieselgel (0,04-0,063 mm, 300 g, Schichthöhe: 30 cm, Schichtdurchmesser: 6 cm) und Aethylacetat als Elutionsmittel unter einem Druck von 0,2 atü sowie einer Dauer von ca. 1 Stunde unterzogen wird. Es werden 60 Fraktionen zu 20-30 ml gesammelt. Die das reine Produkt enthaltenden Fraktionen 21-30 lieferten nach dem Eindampfen und Fällen aus Aethylacetat/Petroläther weisse,reine, amorphe Titelsubstanz mit $[\alpha]_D^{25}$ = -133,5° (c = 1,07 in Aceton). Die Mikroanalyse und das Kernresonanzspektrum entsprechen der angegebenen Struktur.

Das oben verwendete Natriumsalz der (6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carbonsäure kann wie folgt hergestellt werden:

9,08 g (6R,7R)-7-[4-Brom-2-(Z)-methoxyimino-3-oxobutyramido]-3-[[[5-(äthoxycarbonyl)-2-methyl-2H-1,2,4-triazol-3-yl]thio]methyl]-8-oxo-5-thia-1-azabicyclo[4.2.0]oct-2-en-carbonsäure (hergestellt in Analogie zu Beispiel 1) werden in 200 ml Aethanol gelöst und mit 2,25 g Thioharnstoff versetzt. Das Gemisch wird 1 Stunde bei 20°C gerührt, und es werden 300 ml Methanol sowie 2,5 g Aktivkohle zugegeben. Das Gemisch wird weitere 30 Minuten bei 20°C gerührt. Zwecks Abtrennung der Kohle wird es durch einen

Faltenfilter filtriert. Das Filtrat wird mit 500 ml Aethanol versetzt und im Vakuum bei 40°C stark eingeengt. Das dabei ausgefallene Material wird abgenutscht, mit Aethanol, Aether und tiefsiedendem Petroläther nacheinander gewaschen und über Nacht im Hochvakuum bei 45°C getrocknet. Man erhält reine, beigefarbene Titelsubstanz mit $[\alpha]_D^{25}$ = -31° (c = 1 in Wasser). Die Mikroanalyse und das Kernresonanzspektrum entsprechen der angegebenen Struktur.

## Beispiel 3

Herstellung von Methylen-(6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carboxylat-pivalat

6,04 g Natriumsalz der (6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carbonsäure werden in 100 ml Dimethylformamid gelöst und bei 0-5°C mit 4,48 g Pivaloyloxymethyljodid versetzt. Das Gemisch wird 1 Stunde bei 0-5°C unter Stickstoff-Begasung gerührt, dann auf 500 ml Wasser gegossen und 2 mal mit je 300 ml Aethylacetat extrahiert. Die vereinigten organischen Phasen werden nacheinander 2 mal mit 250 ml Wasser, 2 mal mit 250 ml 5%iger Natriumhydrogencarbonat-Lösung und nochmals mit 250 ml Wasser gewaschen, über Natriumsulfat getrocknet und im Vakuum bei 40°C stark eingeengt. Nach Zugabe von tiefsiedendem Petroläther fällt das Rohprodukt aus. Man erhält eine beigefarbene, amorphe Substanz, welche, zwecks Reinigung, einer Flash-Chromatographie an einer Säule mit Kieselgel (0,04-0,063 mm, 300 g, Schichthöhe: 30 cm, Schichtdurchmesser: 6 cm) und Aethylacetat als Elutionsmittel unter einem Druck von 0,1-0,2 atü während ca. 1 Stunde unterzogen wird. Die das reine Produkt enthaltenden Fraktionen liefern nach dem Eindampfen und Fällen aus Aethylacetat/tiefsiedendem Petroläther weisse, amorphe Titel-

substanz mit $[\alpha]_D^{25}$ = -131° (c = 0,92 in Aceton). Die Mikroanalyse und das Kernresonanzspektrum entsprechen der angegebenen Struktur.

Das oben verwendete Natriumsalz der (6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carbonsäure kann wie folgt hergestellt werden:

a) Herstellung von (6R,7R)-7-Amino-3-[[[2-äthyl-5-(methoxycarbonyl)-2H-1,2,4-triazol-3-yl]thio]methyl]-8-oxo-5-thia-1-azabicyclo[4.2.0]oct-2-en-2-carbonsäure

13,6 g 7-Aminocephalosporansäure werden in 125 ml Acetonitril zusammen mit 10,3 g 1-Aethyl-4,5-dihydro-5-thioxo-1H-1,2,4-triazol-3-carbonsäure-methylester (hergestelle in Analogie zu Beispiel 1) und 21,3 g Bortrifluoridätherat 1 Stunde bei 50-55°C unter Stickstoff-Begasung und Feuchtigkeitsausschluss gerührt. Das Gemisch wird auf 20°C gekühlt, 250 ml Wasser werden zugegeben und das pH wird mit konz. Ammoniak auf 3,5 gestellt. Nach halbstündigem Rühren bei 0-5°C wird die ausgefallene Substanz abgenutscht, mit 250 ml Wasser, 250 ml Methanol, 250 ml Aceton und 250 ml tiesiedendem Petroläther gewaschen und über Nacht im Hochvakuum bei 45°C getrocknet. Man erhält reine Titelsubstanz, deren Kernresonanzspektrum im Einklang mit der angegebenen Struktur steht.

b) Herstellung von 2-(2-Amino-thiazol-4-yl)-2-(Z)-methoxyimino-essigsäure-benzthiazol-2-thioester

64,8 g 2-(2-Amino-4-thiazolyl)-2-(Z)-methoxyimino-essigsäure (wasserfrei, enthaltend etwa 5% Methanol) und 112,5 g 2',2-Dithio-bis-benzothiazol werden in 1,8 l Acetonitril suspendiert und unter Rühren mit 40,4 ml N-Methylmorpholin versetzt. Die erhaltene Suspension wird auf 30°C erwärmt und innerhalb von 60 Minuten mit 64,8 ml

Triäthylphosphit versetzt. Die Suspension wird anschliessend 60 Minuten bei 30°C und dann 60 Minuten bei 0°C gerührt. Das Produkt wird abgenutscht, mit Acetonitril und dann mit Aether gewaschen und im Vakuum bei 35°C getrocknet. Man erhält die Titelsubstanz vom Fp. 140°C .

c) Herstellung des Natriumsalzes der (6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carbonsäure

8,99 g (6R,7R)-7-Amino-3-[[[2-äthyl-5-(methoxycarbonyl)-2H-1,2,4-triazol-3-yl]thio]methyl]-8-oxo-5-thia-1-azabicyclo[4.2.0]oct-2-en-2-carbonsäure werden in 225 ml Dichlormethan suspendiert und mit 3,15 ml Triäthylamin versetzt, wobei sofort eine Lösung entsteht. Zu dieser Lösung werden 11,8 g 2-(2-Amino-thiazol-4-yl)-2-(Z)-methoxyimino-essigsäure-benzthiazol-2-thioester zugegeben. Das Gemisch wird 3 Stunden bei 20°C gerührt. Die dunkle Lösung wird zur Abtrennung von wenig Ungelöstem durch einen Faltenfilter filtriert. Das Filtrat wird mit 1,43 ml Methansulfonsäure versetzt, wobei die Cephalosporinsäure jedoch nicht ausfällt. Nach Zugabe von 30 ml einer 2N Lösung von 2-Aethylcapronsäure-Natriumsalz in Essigester fällt das Cephalosporin als Natriumsalz aus, ist jedoch nicht nutschbar. Nun werden 600 ml Methanol und 600 ml Aethanol zugegeben, wobei eine Lösung entsteht. Nach Zugabe von 5 g Aktivkohle wird das Gemisch 30 Minuten bei 20°C gerührt. Nach dem Filtrieren durch einen Faltenfilter wird das Filtrat im Vakuum bei 40°C stark eingeengt. Nach mehrmaligem Abdampfen mit Aethanol wird das ausgefallene Material abgenutscht, mit Aethanol und tiefsiedendem Petroläther gewaschen und über Nacht im Vakuum bei 40°C getrocknet. Man erhält ein Rohprodukt, welches ausser der Titelsubstanz noch 2-(2-Amino-4-thiazolyl)-2-(Z)-methoxyimino-essigsäure-Natriumsalz enthält. Zur Reinigung wird dieses Rohprodukt in 200 ml Methanol gelöst. Die Lösung wird mit 800 ml Aethanol verdünnt und am Vakuum

bei 40°C stark eingeengt. Die dabei ausgefallene Substanz wird abgenutscht, mit Aethanol, Aether und tiefsiedendem Petroläther gewaschen und über Nacht im Hochvakuum bei 45°C getrocknet. Man erhält reine Titelsubstanz mit $[\alpha]_D^{25}$ = -31,9° (c = 1,0 in Wasser), welche nach Mikroanalyse und Kernresonanzspektrum ca. 1 Mol Methansulfonsäure-Natriumsalz und 1/4 Mol Aethanol enthält.

## Beispiel 4

Herstellung von Methylen-(6R,7R)-7-[(Z)-2-(2-Amino-4-thiazolyl)-2-(methoxyimino)acetamido]-3-[[[5-(methoxycarbonyl)-2-butyl-2H-1,2,4-triazol-3-yl]thio]methyl]-8-oxo-5-thia-1-azabicyclo[4.2.0]oct-2-en-2-carboxylat-pivalat

6,32 g Natriumsalz der (6R,7R)-7-[(Z)-2-(2-Amino-4-thiazolyl)-2-(methoxyimino)acetamido]-3-[[[5-(methoxycarbonyl)-2-butyl-2H-1,2,4-triazol-3-yl]thio]methyl]-8-oxo-5-thia-1-azabicyclo[4.2.0]oct-2-en-2-carbonsäure werden in 100 ml Dimethylformamid gelöst und bei 0-5°C mit 4,48 g Pivaloyloxymethyljodid versetzt. Das Gemisch wird 1 Stunde bei 0-5°C unter Stickstoff-Begasung gerührt, auf 500 ml Wasser gegossen und 2mal mit je 300 ml Aethylacetat extrahiert. Die vereinigten Aethylacetat-Extrakte werden nacheinander mit je 2mal 250 ml Wasser, 2mal 250 ml 5%iger wässriger Natriumhydrogencarbonat-Lösung und nochmals mit 250 ml Wasser gewaschen, über Natriumsulfat getrocknet und im Vakuum bei 40°C stark eingeengt. Nach Zugabe von tiefsiedendem Petroläther wird das ausgefallene Rohprodukt abgenutscht, mit tiefsiedendem Petroläther gewaschen und über Nacht im Vakuum bei 25°C getrocknet. Man erhält ein Rohprodukt das mittels Flash-Säulenchromatographie an Kieselgel mit Aethylacetat als Elutionsmittel gereinigt wird. Man erhält nach Ausfällung aus Aethylacetat mit tiefsiedendem Petroläther weisse, reine, amorphe Titelsubstanz mit $[\alpha]_D^{25}$ = -123,8° (c = 0,935 in Aceton). Das Kernresonanzspektrum und die Mikroanalyse entsprechen der angegebenen Struktur.

Das oben verwendete Natriumsalz der (6R,7R)-7-[(Z)-2-(-Amino-4-thiazolyl)-2-(methoxyimino)acetamido]-3-[[[5-(methoxycarbonyl)-2-butyl-2H-1,2,4-triazol-3-yl]thio]methyl]-8-oxo-5-thia-1-azabicyclo[4.2.0]oct-2-en-2-carbonsäure kann wie folgt hergestellt werden:

26,7 g Butylhydrazin-oxalat werden in 500 ml Wasser gelöst und mit 14,55 g Kaliumrhodanid und 50 ml 3N Natronlauge versetzt, wobei das pH des Gemisches auf ca. 5 steigt. Nach ca. 30 Minuten wird die klare Lösung im Vakuum bei 45°C eingedampft und 1mal mit einem Gemisch von 250 ml Toluol und 250 ml Aethanol abgedampft. Der Eindampfrückstand wird mit 100 ml Aethanol geschüttelt; der Festkörper wird abgenutscht, mit Aethanol gewaschen und verworfen. Das Filtrat und der Waschäthanol werden im Vakuum bei 50°C zur Trockene eingedampft. Der resultierende Sirup wird in 100 ml Toluol 45 Minuten unter Rückflussbedingungen und Wasserabscheidung erhitzt. Nach dem Abkühlen auf 20°C wird das ausgefallene Material abgenutscht und anschliessend aus 150 ml Wasser umkristallisiert sowie im Vakuum bei 50°C getrocknet. Man erhält 2-Butylthiosemicarbazid als weisse Kristalle vom Smp. 90-92°C. Mikroanalyse und Kernresonanzspektrum entsprechen der angegebenen Struktur.

17,1 g (6R,7R)-7-Amino-3-[[[5-(methoxycarbonyl)-2-butyl-2H-1,2,4-triazol-3-yl]thio]methyl]-8-oxo-5-thia-1-azabicyclo[4.2.0]oct-2-en-2-carbonsäure werden in 200 ml Methanol suspendiert und mit 5,57 ml Triäthylamin versetzt. Der entstandenen Lösung werden 15,36 g 2-(2-Aminothiazol-4-yl)-2-(Z)-methoxyimino-essigsäure-benzthiazol-2-thioester hinzugefügt, und das Gemisch wird 2 Stunden bei 20°C gerührt. Nach 30 Minuten ist bereits eine Lösung entstanden. Es werden 5 g Aktivkohle und 50 ml einer 2N Lösung von 2-Aethylcapronsäure-Natriumsalz in Aethylacetat zugeben. Das Gemisch wird darauf 15 Minuten bei 20°C gerührt und durch einen Faltenfilter filtriert. Das noch immer gefärbte Filtrat wird mit Aethanol auf 1500 ml verdünnt und mittels Faltenfilter von wenig amorph ausgefallenem Material abfiltriert. Das Filtrat wird im Vakuum bei 40°C auf ein Volumen von ca. 400 ml eingeengt. Das ausgefallene Material wird abgenutscht, mit Aethanol und tiefsiedendem Petroläther gewaschen und über Nacht im Hochvakuum bei 45°C getrocknet. Man erhält Natriumsalz der (6R,7R)-7-[(Z)-2-(2-Amino-4-thiazolyl)-2-(methoxyimino)acetamido]-3-[[[5-(methoxycarbonyl)-2-butyl-2H-1,2,4-triazol-3-yl]thio]methyl]-8-oxo-5-thia-1-azabicyclo[4.2.0]oct-2-en-2-carbonsäure mit $[\alpha]_D^{25}$ = -39,7° (c = 1 in Wasser), deren Kernresonanzspektrum mit der angegebenen Struktur übereinstimmt. Aus der Mutterlauge kann noch weitere Reinsubstanz gewonnen werden.

37 g 2-Butylthiosemicarbazid werden in 500 ml Acetonitril gelöst und mit 42,3 g Natriumhydrogencarbonat versetzt. Nun wird eine Lösung von 26,25 ml Oxalsäure-monomethylesterchlorid in 50 ml Acetonitril während 30 Minuten bei 50-55°C zugetropft. Das Gemisch wird noch 30 Minuten bei 50-55°C gerührt, auf 20°C gekühlt und von Salzen abfiltriert. Das orangefarbene Filtrat wird im Vakuum bei 40°C eingedampft und gut abgetropft. Man erhält N-(2-Butyl-3-thiosemicarbazido)oxamsäuremethylester als oranges Harz, dessen Kernresonanz- und Infrarotspektrum der angegebenen Struktur entsprechen. Zur Cyclisierung wird eine Lösung von 60 g dieser Verbindung in 300 ml Methanol mit einer Lösung von 5,78 g Natrium in 300 ml Methanol versetzt. Das Gemisch wird dann 30 Minuten unter Rückfluss gekocht, auf 20°C gekühlt und im Vakuum bei 40°C auf ein Volumen von ca. 200 ml eingeengt. Das dabei auskristallisierte Material wird durch Abnutschen abgetrennt. Die Mutterlauge wird im Vakuum bei 45°C zur Trockene eingedampft. Der Eindampfrückstand wird in 250 ml Wasser gelöst und mit 30 ml 25%iger Salzsäure versetzt. Das Gemisch wird 10 Minuten bei 20°C gerührt. Das dabei ausgeschiedene, orange Kristallisat wird abgenutscht, mit wenig Wasser gewaschen und über Nacht im Vakuum bei 40°C getrocknet. Man erhält ein orangefarbenes Rohprodukt, welches zur Reinigung an einer Kieselsäule mit Dichlormethan als Elutionsmittel chromatographiert wird. Die das Hauptprodukt enthaltenden Fraktionen werden

gesammelt und im Vakuum bei 40°C auf ca. 100 ml eingeengt. Nach der Zugabe von tiefsiedendem Petroläther kristallisiert die Substanz aus. Diese wird abgenutscht, mit tiefsiedendem Petroläther gewaschen und über Nacht im Vakuum bei 45°C getrocknet. Man erhält reinen 1-Butyl-4,5-dihydro-5-thioxo-1H-1,2,4-triazol-3-carbonsäure-methylester vom Smp. 106-108°C.

30 g 7-Aminocephalosporansäure werden zusammen mit 26,9 g 1-Butyl-4,5-dihydro-5-thioxo-1H-1,2,4-triazol-3-carbonsäure-methylester in 200 ml Acetonitril suspendiert und mit 120 g Bortrifluorid-acetonitril-Komplex (ca. 19% $BF_3$) versetzt. Das Gemisch wird 1 Stunde bei 50-55°C unter Stickstoff-Begasung und Feuchtigkeitsausschluss gerührt, auf 10°C gekühlt, mit 130 ml Wasser versetzt und mittels konz. Ammoniak auf pH 1,7 gestellt, wobei praktisch keine Substanz ausfällt. Es werden weitere 370 ml Wasser zugegeben und das pH des Gemisches mit konz. Ammoniak auf 3,5 gestellt, wobei die Substanz ausfällt. Nach 15 Minuten Rühren bei 10°C wird letztere abgenutscht, mit 500 ml Wasser, 500 ml Methanol, 500 ml Aceton und 500 ml tiefsiedendem Petroläther gewaschen und über Nacht im Vakuum bei 40°C getrocknet. Man erhält reine (6R,7R)-7-Amino-3-[[[5-(methoxycarbonyl)-2-butyl-2H-1,2,4-triazol-3-yl]thio]methyl]-8-oxo-5-thia-1-azabicyclo[4.2.0]oct-2-en-2-carbonsäure, deren Kernresonanzspektrum mit der angegebenen Struktur übereinstimmt.

## Beispiel 5

In Analogie zu Beispiel 1 erhält man ebenfalls

- Methylen-(6R,7R)-7-[(Z)-2-amino-4-thiazolyl)-2-(methoxyimino)acetamido]-3-[[[5-(methoxycarbonyl)-2-[[[(pivaloyloxy)methoxy]carbonyl]methyl]-2H-1,2,4-triazol-3-yl]thio]methyl]-8-oxo-5-thia-1-azabicyclo[4.2.0]oct-2-en-2-carboxylat-pivalat als weisses, amorphes Pulver mit $[\alpha]_D^{20}$ = -96,4° (c = 0,932 in Aceton).

- Methylen-(6R,7R)-7-[(Z)-2-(2-amino-4-thiazolyl)-2-(methoxyimino)acetamido]-3-[[[5-(methoxycarbonyl)-2-(n-propyl)-2H-1,2,4-triazol-3-yl]thio]methyl]-8-oxo-5-thia-1-azabicyclo[4.2.0]oct-2-en-2-carboxylat-pivalat als weisses, amorphes Pulver mit $[\alpha]_D^{20}$ = -120,4° (c = 1 in Aceton).

- Methylen-(6R,7R)-7-[(Z)-2-(2-amino-4-thiazolyl)-2-(methoxyimino)acetamido]-3-[[[5-(methoxycarbonyl)-2-phenyl-2H-1,3,4-triazol-3-yl]thio]methyl]-8-oxo-5-thia-1-azabicyclo[4.2.0]oct-2-en-2-carboxylat-pivalat als weisses, amorphes Pulver mit $[\alpha]_D^{20}$ = -83,5° (c = 1.043 in Aceton).

Die Kernresonanzspektren und die Mikroanalysen dieser Verbindungen entsprechen den angegebenen Strukturen.

## Beispiel 6

Es wird in üblicher Weise eine Gelatine-Steckkapsel folgender Zusammensetzung hergestellt:

| | |
|---|---|
| Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carboxylat-pivalat | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

## Beispiel 7

Es wird in üblicher Weise eine Tablette folgender Zusammensetzung hergestellt:

| | |
|---|---|
| Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-triazol-3-yl]thio]methyl]-8-oxo-5-thia-1-azabicyclo[4.2.0]oct-2-en-2-carboxylat-pivalat | 250 mg |
| Lactose | 70 mg |
| Maisstärke | 65 mg |
| Polyvinylpyrrolidon | 10 mg |
| Magnesiumstearat | 5 mg |
| | 400 mg |

Mit dem Wirkstoff, der Lactose, dem Polyvinylpyrrolidon und 40 Gewichtsteilen der Maisstärke wird in üblicher Weise ein Granulat hergestellt. Dieses wird mit den restlichen 25 Gewichtsteilen Maisstärke und 5 Gewichtsteilen Magnesiumstearat vermischt und zu Tabletten gepresst.

## Patentansprüche

1. Leicht hydrolysierbare Ester von Cephalosporinderivaten der allgemeinen Formel

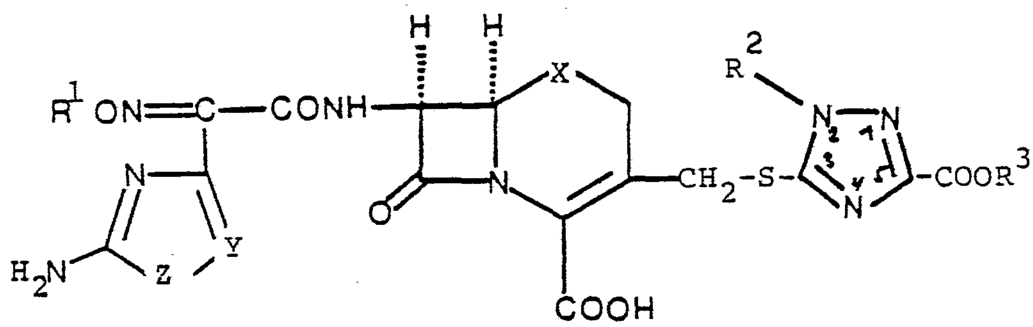

I

in der $R^1$ Wasserstoff, Methyl oder Carboxy-niederes Alkyl, X Schwefel, Sauerstoff oder eine der Gruppen -SO- und $-SO_2-$, Y die Gruppe -CH= oder Stickstoff, Z Schwefel oder Selen, $R^2$ gegebenenfalls durch Carboxy, Hydroxy, leicht hydrolysierbares Acyloxy oder Dimethylamino substituiertes niederes Alkyl oder Phenyl und $R^3$ niederes Alkyl, Phenyl-$C_{2-4}$-alkyl, $R^4$-Phenyl-niederes Alkyl, worin $R^4$ Halogen, niederes Alkyl oder niederes Alkoxy darstellt, bedeutet,

sowie Säureadditionssalze dieser Ester und Hydrate dieser Ester bzw. Säureadditionssalze.

2. Ester gemäss Anspruch 1, dadurch gekennzeichnet, dass $R^2$ gegebenenfalls durch Carboxy, Hydroxy, leicht hydrolysierbares Acyloxy oder Dimethylamino substituiertes niederes Alkyl darstellt.

3. Ester nach Anspruch 2 in der syn-isomeren Form bzw. Gemische, in denen die syn-isomere Form überwiegt.

4. Ester nach Anspruch 2 oder 3, dadurch gekennzeichnet, dass $R^2$ n-Butyl, Hydroxymethyl, Dimethylaminomethyl oder Pivaloyloxymethyl darstellt.

5. Ester nach einem der Ansprüche 2-4, dadurch gekennzeichnet, dass X Schwefel darstellt.

6. Ester nach einem der Ansprüche 2-4, dadurch gekennzeichnet, dass X die Gruppe -SO- darstellt.

7. Ester nach einem der Ansprüche 2-6, dadurch gekennzeichnet, dass Y die Gruppe -CH= darstellt.

8. Ester nach einem der Ansprüche 2-6, dadurch gekennzeichnet, dass Y Stickstoff darstellt.

9. Ester nach einem der Ansprüche 2-8, dadurch gekennzeichnet, dass Z Schwefel darstellt.

10. Ester nach einem der Ansprüche 2-9, dadurch gekennzeichnet, dass $R^1$ Wasserstoff darstellt.

11. Ester nach einem der Ansprüche 2-9, dadurch gekennzeichnet, dass $R^1$ Methyl darstellt.

12. Ester nach einem der Ansprüche 2-9, dadurch gekennzeichnet, dass $R^1$ Carboxymethyl darstellt.

13. Ester nach einem der Ansprüche 2-9, dadurch gekennzeichnet, dass $R^1$ 1-Carboxy-1-methyl-äthyl darstellt.

14. Ester nach einem der Ansprüche 2-13, dadurch gekennzeichnet, dass $R^3$ Methyl darstellt.

15. Ester nach einem der Ansprüche 2-13, dadurch gekennzeichnet, dass $R^3$ Aethyl darstellt.

16. Ester nach einem der Ansprüche 2-15, dadurch gekennzeichnet, dass $R^2$ Methyl darstellt.

17. Ester nach einem der Ansprüche 2-15, dadurch gekennzeichnet, dass $R^2$ Aethyl darstellt.

18. Ester nach einem der Ansprüche 2-15, dadurch gekennzeichnet, dass $R^2$ n-Propyl darstellt.

19. Ester nach einem der Ansprüche 2-15, dadurch gekennzeichnet, dass $R^2$ Carboxy-methyl darstellt.

20. Pivaloyloxymethylester gemäss einem der Ansprüche 2-19.

21. Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-thiazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

22. Methylen-(6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

23. Methylen (6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

24. Methylen-(6R,7R)-7-[(Z)-2-(2-Amino-4-thiazolyl)-2-(methoxyimino)-acetamido]-3-[[[5-(methoxycarbonyl)-2-[[[(pivaloyloxy)methoxy]carbonyl]methyl]-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

25. Methylen-(6R,7R)-7-[(Z)-2-(2-Amino-4-thiazolyl)-2-(methoxyimino)-acetamido]-3-[[[5-(methoxycarbonyl)-2-(n-propyl)-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionssalze.

26. Methylen-(6R,7R)-7-[(Z)-2-(2-Amino-4-thiazolyl)-2-(methoxyimino)-acetamido]-3-[[[5-(methoxycarbonyl)-2-phenyl-2H-1,2,4-triazol-3-yl]thio]methyl]-8-oxo-5-thia-1-azabicyclo[4.2.0]oct-2-en-2-carboxylat-pivalat sowie Säureadditionssalze dieser Verbindung und Hydrate dieser Verbindung bzw. Säureadditionsalze.

27. Carbonsäuren der allgemeinen Formel

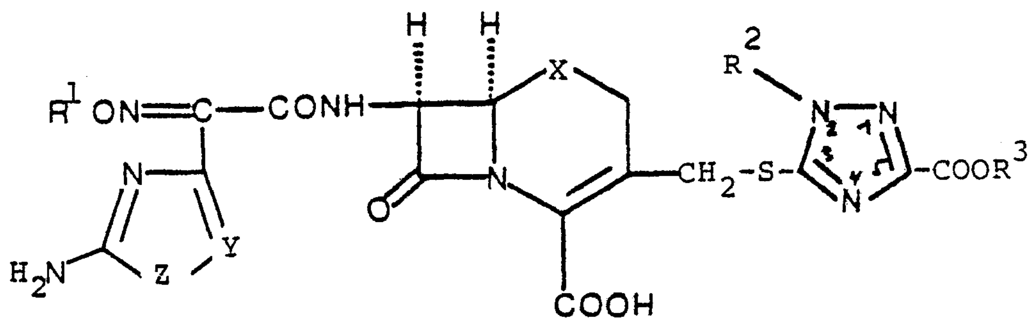

I

in der $R^1$, $R^2$, $R^3$, $R^4$, X und Y die in Anspruch 1 gegebene Bedeutung haben,

sowie Salze dieser Verbindungen und Hydrate dieser Verbindungen bzw. Salze.

28. Verbindungen der allgemeinen Formel

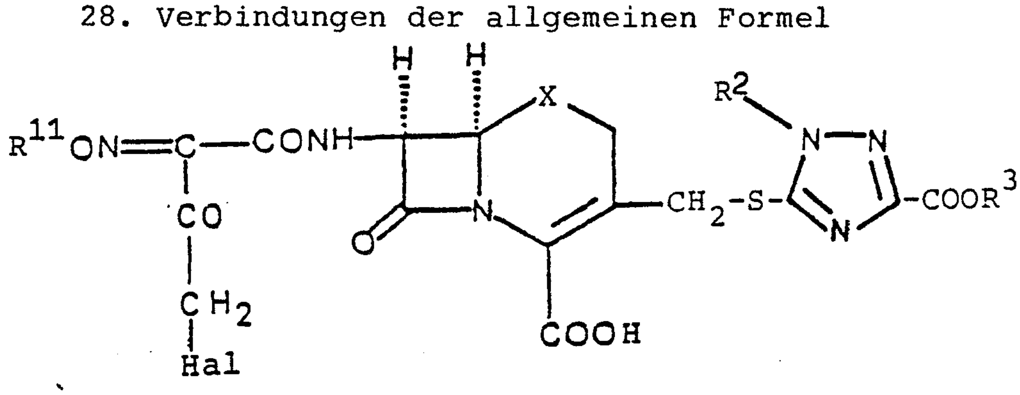

II

in der $R^2$, $R^3$ und X die in Anspruch 1 gegebene Bedeutung haben, $R^{11}$ Wasserstoff oder Methyl, Hal ein Halogenatom darstellt und die Carboxygruppe in geschützter Form vorliegen kann,

sowie Salze dieser Verbindungen.

29. Verbindungen der allgemeinen Formel

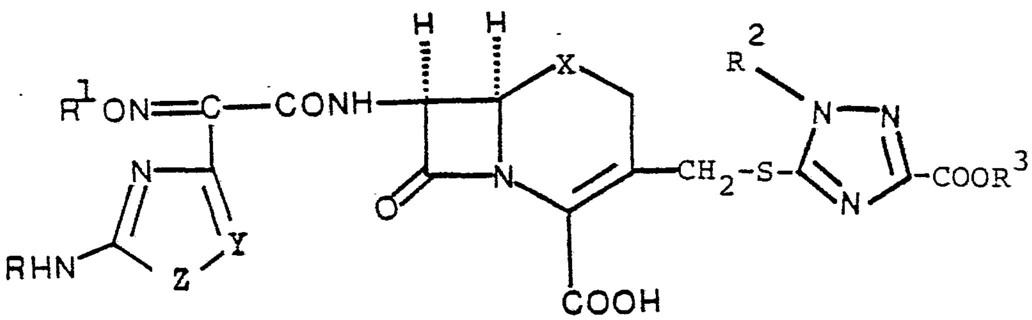

III

in der $R^1$, $R^2$, $R^3$, X, Y und Z die in Anspruch 1 gegebene Bedeutung haben, R eine abspaltbare Schutzgruppe darstellt und die Carboxygruppe in geschützter Form vorliegen kann,

sowie Salze dieser Verbindungen.

30. Verbindungen der tautomeren Formeln

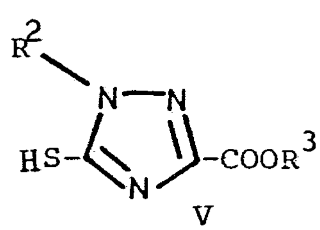

V

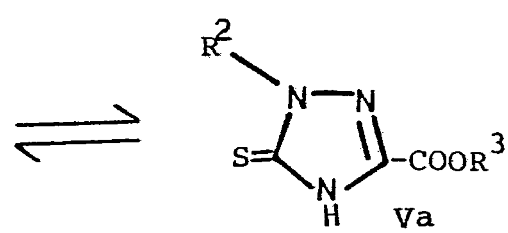

Va

in denen $R^2$ und $R^3$ die in Anspruch 1 gegebene Bedeutung haben.

31. Verbindungen der allgemeinen Formel

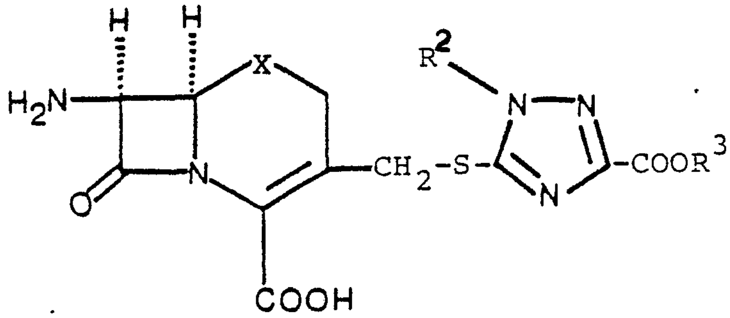

VI

in der X, $R^2$ und $R^3$ die in Anspruch 1 gegebene Bedeutung haben und die Carboxygruppe und/oder Aminogruppe in geschützter Form vorliegen kann.

32. Verbindungen der allgemeinen Formel

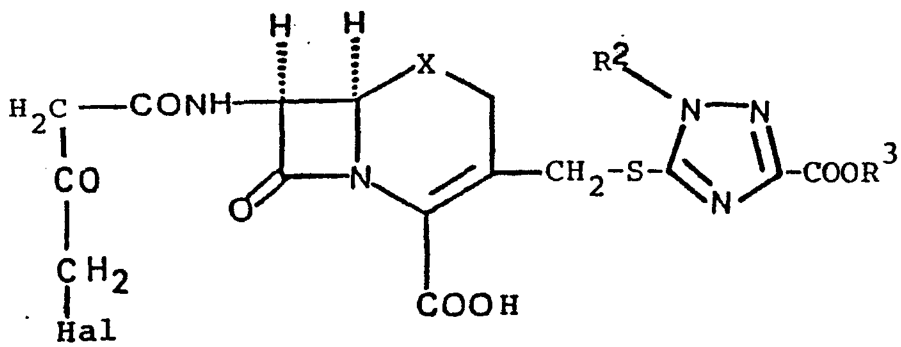

XVI

in der X, $R^2$ und $R^3$ die in Anspruch 1 gegebene Bedeutung haben und Hal ein Halogenatom darstellt.

33. Verbindungen gemäss einem der Ansprüche 1-27 als pharmazeutische Wirkstoffe.

34. Verbindungen gemäss einem der Ansprüche 1-26 als pharmazeutische Wirkstoffe zur enteralen, z.B. oralen, Behandlung und Prophylaxe von Infektionskrankheiten.

35. Pharmazeutische Präparate, gekennzeichnet durch einen Gehalt an einer Verbindung gemäss einem der Ansprüche 1-27.

36. Pharmazeutische Präparate zur enteralen, z.B. oralen, Behandlung und Prophylaxe von Infektionskrankheiten, gekennzeichnet durch einen Gehalt an einer Verbindung gemäss einem der Ansprüche 1-26.

37. Verfahren zur Herstellung der Verbindungen gemäss einem der Ansprüche 1-26, dadurch gekennzeichnet, dass man die entsprechende Carbonsäure, d.h. eine Verbindung der Formel I oder ein Salz dieser Verbindung, einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

38. Verfahren nach Anspruch 37, dadurch gekennzeichnet, dass man als veresterndes Mittel ein Pivaloyloxymethylhalogenid, insbesondere das Jodid, einsetzt.

39. Verwendung von Verbindungen gemäss einem der Ansprüche 1-27 bei der Behandlung bzw. Prophylaxe von Krankheiten.

40. Verwendung von Verbindungen gemäss einem der Ansprüche 1-27 bei der Behandlung bzw. Prophylaxe von Infektionskrankheiten.

41. Verwendung von Verbindungen gemäss einem der Ansprüche 1-26 bei der enteralen, z.B. oralen, Behandlung bzw. Prophylaxe von Infektionskrankheiten.

***

## Patentansprüche für OESTERREICH

1. Verfahren zur Herstellung von leicht hydrolysierbaren Estern von Cephalosporinderivaten der allgemeinen Formel

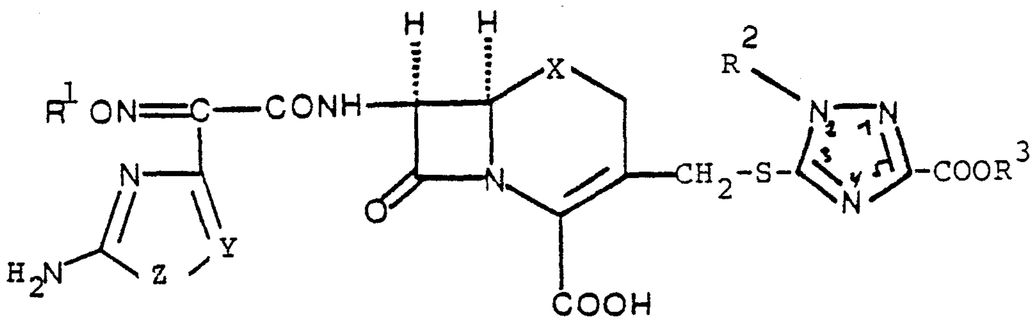

I

in der $R^1$ Wasserstoff, Methyl oder Carboxy-niederes Alkyl, X Schwefel, Sauerstoff oder eine der Gruppen -SO- und $SO_2$-, Y die Gruppe -CH= oder Stickstoff, Z Schwefel oder Selen, $R^2$ gegebenenfalls durch Carboxy, Hydroxy, leicht hydrolysierbares Acyloxy oder Dimethylamino substituiertes niederes Alkyl oder Phenyl und $R^3$ niederes Alkyl, Phenyl-$C_{2-4}$-alkyl, $R^4$-Phenyl-niederes Alkyl, worin $R^4$ Halogen, niederes Alkyl oder niederes Alkoxy darstellt, bedeutet,

sowie von Säureadditionssalzen dieser Ester und von Hydraten dieser Ester bzw. Säureadditionssalze, dadurch gekennzeichnet, dass man die entsprechende Carbonsäure, d.h. eine Verbindung der Formel I oder ein Salz dieser Verbindung, einer entsprechenden Veresterung unterwirft, erwünschtenfalls ein erhaltenes Produkt S-oxidiert und erwünschtenfalls das erhaltene Produkt in ein Säureadditionssalz oder Hydrat bzw. ein Hydrat dieses Säureadditionssalzes überführt.

2. Verfahren nach Anspruch 1 zur Herstellung von Estern gemäss Anspruch 1, worin $R^2$ gegebenenfalls durch Carboxy, Hydroxy, leicht hydrolysierbares Acyloxy oder Dimethylamino substituiertes niederes Alkyl darstellt,

dadurch gekennzeichnet, dass man entsprechend substituierte Ausgangsverbindungen umsetzt.

3. Verfahren nach Anspruch 2 zur Herstellung von Estern nach Anspruch 2 in der syn-isomeren Form bzw. von Gemischen, in denen die syn-isomere Form überwiegt, dadurch gekennzeichnet, dass man eine Ausgangsverbindung mit entsprechender Konfiguration einsetzt oder ein erhaltenes syn/anti-Gemisch auftrennt.

4. Verfahren nach Anspruch 2 oder 3 zur Herstellung von Estern nach Anspruch 2 oder 3, worin $R^2$ n-Butyl, Hydroxymethyl, Dimethylaminomethyl oder Pivaloyloxymethyl darstellt, dadurch gekennzeichnet, dass man entsprechend substituierte Ausgangsverbindungen einsetzt.

5. Verfahren nach einem der Ansprüche 2-4 zur Herstellung von Estern nach einem der Ansprüche 2-4, worin X Schwefel darstellt, dadurch gekennzeichnet, dass man entsprechend substituierte Ausgangsverbindungen einsetzt.

6. Verfahren nach einem der Ansprüche 2-4 zur Herstellung von Estern nach einem der Ansprüche 2-4, worin X die Gruppe -SO- darstellt, dadurch gekennzeichnet, dass man entsprechend substituierte Ausgangsverbindungen einsetzt.

7. Verfahren nach einem der Ansprüche 2-6 zur Herstellung von Estern nach einem der Ansprüche 2-6, worin Y die Gruppe -CH= darstellt, dadurch gekennzeichnet, dass man entsprechend substituierte Ausgangsverbindungen einsetzt.

8. Verfahren nach einem der Ansprüche 2-6 zur Herstellung von Estern nach einem der Ansprüche 2-6, worin Y Stickstoff darstellt, dadurch gekennzeichnet, dass man entsprechend substituierte Ausgangsverbindungen einsetzt.

9. Verfahren nach einem der Ansprüche 2-8 zur Herstellung von Estern nach einem der Ansprüche 2-8, worin Z Schwefel darstellt, dadurch gekennzeichnet, dass man entsprechend substituierte Ausgangsverbindungen einsetzt.

10. Verfahren nach einem der Ansprüche 2-9 zur Herstellung von Estern nach einem der Ansprüche 2-9, worin $R^1$ Wasserstoff darstellt, dadurch gekennzeichnet, dass man entsprechend substituierte Ausgangsverbindungen einsetzt.

11. Verfahren nach einem der Ansprüche 2-9 zur Herstellung von Estern nach einem der Ansprüche 2-9, worin $R^1$ Methyl darstellt, dadurch gekennzeichnet, dass man entsprechend substituierte Ausgangsverbindungen einsetzt.

12. Verfahren nach einem der Ansprüche 2-9 zur Herstellung von Estern nach einem der Ansprüche 2-9, worin $R^1$ Carboxymethyl darstellt, dadurch gekennzeichnet, dass man entsprechend substituierte Ausgangsverbindungen einsetzt.

13. Verfahren nach einem der Ansprüche 2-9 zur Herstellung von Estern nach einem der Ansprüche 1-9, worin $R^1$ 1-Carboxy-1-methyl-äthyl darstellt, dadurch gekennzeichnet, dass man entsprechend substituierte Ausgangsverbindungen einsetzt.

14. Verfahren nach einem der Ansprüche 2-13 zur Herstellung von Estern nach einem der Ansprüche 2-13, worin $R^3$ Methyl darstellt, dadurch gekennzeichnet, dass man entsprechend substituierte Ausgangsverbindungen einsetzt.

15. Verfahren nach einem der Ansprüche 2-13 zur Herstellung von Estern nach einem der Ansprüche 2-13, worin $R^3$ Aethyl darstellt, dadurch gekennzeichnet, dass man entsprechend substituierte Ausgangsverbindungen einsetzt.

16. Verfahren nach einem der Ansprüche 2-15 zur Herstellung von Estern nach einem der Ansprüche 2-15, worin $R^2$ Methyl darstellt, dadurch gekennzeichnet, dass man entsprechend substituierte Ausgangsverbindungen einsetzt.

17. Verfahren nach einem der Ansprüche 2-15 zur Herstellung von Estern nach einem der Ansprüche 2-15, worin $R^2$ Aethyl darstellt, dadurch gekennzeichnet, dass man entsprechend substituierte Ausgangsverbindungen einsetzt.

18. Verfahren nach einem der Ansprüche 2-15 zur Herstellung von Estern nach einem der Ansprüche 2-15, worin $R^2$ n-Propyl darstellt, dadurch gekennzeichnet, dass man entsprechend substituierte Ausgangsverbindungen einsetzt.

19. Verfahren nach einem der Ansprüche 2-15 zur Herstellung von Estern nach einem der Ansprüche 2-15, worin $R^2$ Carboxy-methyl darstellt, dadurch gekennzeichnet, dass man entsprechend substituierte Ausgangsverbindungen einsetzt.

20. Verfahren nach einem der Ansprüche 2-19 zur Herstellung von Pivaloyloxymethylestern gemäss einem der Ansprüche 2-19, dadurch gekennzeichnet, dass man als veresterndes Mittel ein Pivaloyloxymethylhalogenid, insbesondere das Jodid, einsetzt.

21. Verfahren nach einem der Ansprüche 1-3, 5, 7, 9, 11, 14, 16 und 20 zur Herstellung von Methylen-(6R,7R)-7-[(Z)-2-(2-Amino-4-thiazolyl)-2-(methoxyimino)acetamido]-3-[[[5-(methoxycarbonyl)-2-methyl-2H-1,2,4-thiazol-3-yl]thio]methyl]-8-oxo-5-thia-1-azabicyclo[4.2.0]oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.

22. Verfahren nach einem der Ansprüche 1-3, 5, 7, 9, 11, 15, 16 und 20 zur Herstellung von Methylen-(6R,7R)-7-[(Z)-2-(2-Amino-4-thiazolyl)-2-(methoxyimino)acetamido]-3-[[[5-(äthoxycarbonyl)-2-methyl-2H-1,2,4-triazol-3-yl]thio]methyl]-8-oxo-5-thia-1-azabicyclo[4.2.0]oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.

23. Verfahren nach einem der Ansprüche 1-3, 5, 7, 9, 11, 14, 17 und 20 zur Herstellung von Methylen (6R,7R)-7-[(Z)-2-(2-Amino-4-thiazolyl)-2-(methoxyimino)acetamido]-3-[[[2-äthyl-5-(methoxycarbonyl)-2H-1,2,4-triazol-3-yl]thio]-methyl]-8-oxo-5-thia-1-azabicyclo[4.2.0]oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.

24. Verfahren nach einem der Ansprüche 1-3, 5, 7, 9, 11, 14, 19 und 20 zur Herstellung von Methylen-(6R,7R)-7-[(Z)-2-(2-Amino-4-thiazolyl)-2-(methoxyimino)acetamido]-3-[[[5-(methoxycarbonyl)-2-[[[(pivaloyloxy)methoxy]carbonyl]-methyl]-2H-1,2,4-triazol-3-yl]thio]methyl]-8-oxo-5-thia-1-azabicyclo[4.2.0]oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.

25. Verfahren nach einem der Ansprüche 1-3, 5, 7, 9, 11, 14, 18 und 20 zur Herstellung von Methylen-(6R,7R)-7-[(Z)-2-(2-Amino-4-thiazolyl)-2-(methoxyimino)acetamido]-3-[[[5-(methoxycarbonyl)-2-(n-propyl)-2H-1,2,4-triazol-3-yl]thio]-

methyl]-8-oxo-5-thia-1-azabicyclo[4.2.0]-oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.

26. Verfahren nach einem der Ansprüche 1-3, 5, 7, 9, 11, 14 und 20 zur Herstellung von Methylen-(6R,7R)-7-[(Z)-2-(2-Amino-4-thiazolyl)-2-(methoxyimino)acetamido]-3-[[[5-(methoxycarbonyl)-2-phenyl-2H-1,2,4-triazol-3-yl]thio]-methyl]-8-oxo-5-thia-1-azabicyclo[4.2.0]oct-2-en-2-carboxylat-pivalat sowie von Säureadditionssalzen dieser Verbindung und von Hydraten dieser Verbindung bzw. Säureadditionssalze, dadurch gekennzeichnet, dass man entsprechend substituierte Ausgangsverbindungen einsetzt.